# EUROPÄISCHE PATENTSCHRIFT

(11) **EP 3 042 694 B1**
(45) Veröffentlichungstag und Bekanntmachung des Hinweises auf die Patenterteilung: **08.01.2020**
(21) Anmeldenummer: 15195554.9
(22) Anmeldetag: 20.11.2015
(51) Int. Cl.: A61N 1/375, A61N 1/372

(54) **EPIKARDIALER HERZSTIMULATOR**
EPICARDIAL HEART STIMULATOR
STIMULATEUR CARDIAQUE ÉPICARDIQUE

(30) Priorität: 06.01.2015 US 201562100099 P
(43) Veröffentlichungstag der Anmeldung: 13.07.2016
(73) Patentinhaber: BIOTRONIK SE & Co. KG, 12359 Berlin (DE)
(72) Erfinder: Blömer, Frank, 14169 Berlin (DE); Moß, Christian, 10589 Berlin (DE)
(74) Vertreter: Galander, Marcus

(56) Entgegenhaltungen:
- US-A1- 2006 085 041
- US-A1- 2007 179 540
- US-A1- 2009 088 813
- US-A1- 2013 079 861
- US-A1- 2014 058 494

## Beschreibung

Die Erfindung betrifft einen epikardialen Herzstimulator zur Stimulation einer Kammer oder Vorkammer, insbesondere zur Stimulation des rechten oder des linken Ventrikels eines Herzens.

Implantierbare Herzstimulatoren in Form von Herzschrittmachern sind grundsätzlich bekannt. Solche Herzstimulatoren sind in der Regel an Elektrodenleitungen angeschlossen, die in einer Kammer eines Herzens oder in unmittelbarer Nähe Stimulations- oder Defibrillationselektroden besitzen. Epikardiale Herzschrittmacher benötigen solche Elektrodenleitungen nicht, sondern werden an der Außenseite eines Herzens implantiert und stimulieren das Herzgewebe (Myokard) über eine an einem Gehäuse angebrachte Stimulationselektrode. Ein solcher epikardialer Herzschrittmacher mit einer Stimulationselektrode in Form einer Helix ist in WO 2013/152259 beschrieben.

Über die Stimulationselektrode kann ein Herzschrittmacher einen elektrischen Stimulationsimpuls an das Muskelgewebe (Myokard) einer Herzkammer abgeben, um so eine stimulierte Kontraktion der Herzkammer hervorzurufen, sofern der Stimulationsimpuls eine ausreichende Intensität besitzt und das Herzmuskelgewebe (Myokard) sich nicht gerade in einer refraktären Phase befindet. Um auf diese Weise eine stimulierte Kontraktion einer Herzkammer auszulösen, werden üblicher Weise relativ kleinflächige Stimulationselektroden verwendet, da es zum Auslösen einer stimulierten Kontraktion einer Herzkammer ausreicht, wenn nur ein kleiner Teil des Myokards dieser Herzkammer anfänglich stimuliert wird. Eine derartige stimulierte Kontraktion einer Herzkammer wird im Rahmen dieser Beschreibung als stimuliertes Ereignis bezeichnet. Kommt es zu einer natürlichen Kontraktion der Herzkammer, wird dies im Rahmen dieser Beschreibung als Eigenaktion oder als intrinsisches Ereignis bezeichnet. Eine Kontraktion beispielsweise des rechten Atriums eines Herzens wird als atriales Ereignis bezeichnet, welches beispielsweise ein natürliches atriales Ereignis sein kann, oder - im Falle eines atrialen Herzschrittmachers - auch ein stimuliertes atriales Ereignis. Im gleichen Sinne können natürliche (intrinsische) und stimulierte linksventrikuläre und rechtsventrikuläre Ereignisse unterschieden werden.

In Bezug auf die Stimulation einer Herzkammer, insbesondere des rechten oder des linken Ventrikels eines Herzens, ist weiter anzumerken, dass diese vorzugsweise atriumsynchron erfolgen, um möglichst gut die natürliche Kontraktionssequenz des Herzens abzubilden, bei der es zunächst zu einer Kontraktion des rechten Atriums und anschließend nach einer atrioventrikulären Überleitungszeit zu einer Kontraktion des rechten Ventrikels und gleichzeitig oder wenig später des linken Ventrikels kommt. Unter bestimmten Umständen unterbleibt bei einigen Patienten die natürliche Kontraktion des Ventrikels im Nachgang einer natürlichen Kontraktion des Atriums. In typischen Zweikammerschrittmachern wird daher die natürliche Kontraktion des Atriums als intrinsisches atriales Ereignis erfasst und nach einer vorgegebenen Überleitungszeit der rechte und/oder der linke Ventrikel stimuliert.

Das Erfassen solcher natürlichen (intrinsischen) Ereignisse erfolgt durch Abfühlen, also Messen, der elektrischen Potenziale des Myokards der jeweiligen Herzkammer mit Hilfe von Sensingelektroden, die bei herkömmlichen Herzstimulatoren Teil einer entsprechenden Elektrodenleitung sind.

Das Erfassen von intrinsischen Ereignissen im Ventrikel erlaubt es beispielsweise einen ventrikulären Herzstimulator im Demand-Modus zu betreiben, in dem die Abgabe von ventrikulären Stimulationsimpulsen immer dann unterdrückt wird wenn innerhalb eines jeweiligen Zeitfensters ein natürliches ventrikuläres Ereignis erfasst wird. Das Erfassen von intrinsischen Ereignissen im Atrium erlaubt es beispielsweise einem ventrikulären Herzstimulator ventrikuläre Stimulationsimpulse - solange sie nicht unterdrückt werden - atriumsynchron, also nach einer vorgegebenen atrioventrikulären Verzögerungszeit nach Erfassen des jeweiligen atrialen Ereignisses abzugeben.

Die typischen Stimulationsmodi, die sich mit einem Herzstimulator verwirklichen lassen, können als bekannt vorausgesetzt werden (VVI, VDD, DDD usw.), so dass sie hier nicht weiter zu erläutern sind.

Epikardiale Herzstimulatoren können an 4 verschiedenen Positionen implantiert werden, je nachdem, welche Herzkammer oder Vorkammer stimuliert werden soll, also links- oder rechtsventrikulär oder links- oder rechtsatrial.

Die US-Patentanmeldung US 20090088813 A1 beschreibt einen epikardialen Herzschrittmacher, der einen mit Elektroden besetzten transmuralen Katheter aufweist. Die auf dem Katheter angeordneten Elektroden dienen sowohl der Stimulation als auch der Erfassung von Herzsignalen.

Die US-Patentanmeldung US 20070179540 A1 beschreibt einen Herzrythmussensor, der über eine Schraube verfügt, mittels derer er am Epikard befestigt werden kann. Eine Tip- und eine Ringelektrode dienen der Erfassung von Herzsignalen, die über eine Telemetrieeinheit an ein weiteres Implantat übermittelbar sind.

Der Erfindung liegt die Aufgabe zugrunde, möglichst weitere Stimulationsmodi für epikardiale Herzstimulatoren zu erschließen.

Erfindungsgemäß wird diese Aufgabe durch einen epikardialen Herzstimulator nach Anspruch 1 gelöst, der ein Gehäuse und in diesem Gehäuse angeordnete elektrische Komponenten einschließlich einer Stimulationseinheit und einer Stimulationssteuereinheit aufweist, so wie an dem Gehäuse wenigstens eine Stimulationselektrode. Die Stimulationselektrode ist mit der Stimulationseinheit verbunden und diese wiederum mit der Stimulationssteuereinheit. Die Stimulationseinheit ist ausgebildet, elektrische Energie für einen Stimulationsimpuls zur Verfügung zu stellen und auf ein entsprechendes Auslösesignal der Stimulationssteuereinheit hin einen Stimulationsimpuls über die Stimulationselektrode abzugeben. Außerdem ist an dem Gehäuse eine Sensingelektrode zum Abfühlen elektrischer Potentiale vorgesehen, die mit einer Sensingeinheit in dem Gehäuse elektrisch verbunden ist. Die Sensingeinheit ist ihrerseits wiederum mit der Stimulationssteuereinheit verbunden und die Stimulationssteuereinheit ist so konfiguriert, dass sie in einem entsprechenden Betriebsmodus das Abgeben eines jeweiligen Auslösesignals in Abhängigkeit eines Ausgangssignals der Sensingeinheit steuert.

Die Erfinder haben erkannt, dass das Sensing intrinsischer Signale bei einem epikardialen Herzstimulator weitgehend lokal ist und damit nur die Erkennung der Aktivität in einer Kammer möglich ist. Da epikardiale Herzstimulatoren nur lokale, an ihrem Gehäuse angebrachte Elektroden (-pole) aufweisen, sind auch die Sensingelektroden immer der Herzkammer oder Vorkammer benachbart, die stimuliert werden soll. Mit einem derartigen epikardialen Herzstimulator ist üblicherweise nur ein Sensing in der Kammer möglich, der der epikardiale Herzschrittmacher benachbart ist, so dass ein derartiger Herzschrittmacher auch solche Einkammer-Betriebsmodi realisieren kann wie AAI oder VVI.

Mit der vorliegenden Erfindung wird die Erkennung der Aktivität auch in einer entfernten Kammer, also einer anderen als derjenigen Kammer, der der epikardiale Herzschrittmacher benachbart ist, möglich. Bei einem ventrikulären epikardialen Herzschrittmacher ist also auch ein Sensing atrialer Ereignisse und dadurch eine vorhofsynchrone Stimulation z.B. im VDD Modus mit einem einzelnen epikardial platzierten ventrikulären Herzstimulator möglich, ohne dass hierzu ein zweites implantiertes Gerät erforderlich ist.

Erfindungsgemäß ist somit die Integration einer oder mehrerer Sensing-Elektroden im Gehäuse des epikardialen Herzstimulators vorgesehen, wobei die Elektroden vorzugsweise einen räumlich maximalen Abstand einnehmen, damit Fernfeld-Signale aus entfernten Kammern wahrgenommen und für das Timing verwendet werden können.

Vorzugsweise ist die Sensingeinheit mit der Sensingelektrode und der Stimulationselektrode oder einer zweiten Sensingelektrode verbunden und die Stimulationssteuereinheit ausgebildet, das Ausgangssignal der Sensingeinheit hinsichtlich solcher Signalmerkmale auszuwerten, die eine Kontraktion einer entfernten Vorkammer und/oder Herzkammer anzeigen. Das Ausgangssignal der Sensingeinheit ist typischerweise ein Femfeldelektrokardiogramm, so dass die Sensingeinheit eine Fernfeldsensingeinheit ist.

Gemäß einer weiteren bevorzugten Ausführungsvariante sind an dem Gehäuse mehrere Sensingelektroden zum Abfühlen elektrischer Potentiale vorgesehen. Die Sensingeinheit umfasst einen Schalter, mit dem eine oder zwei der Sensingelektroden mit den übrigen zum Aufnehmen eines Elektrokardiogramm ausgebildeten Komponenten der Sensingeinheit umschaltbar elektrisch zu verbinden sind

An dem Gehäuse ist ein indifferenter Elektrodenpol in unmittelbarer Nähe der Stimulationselektrode angeordnet und bildet mit dieser zusammen ein Stimulationselektrodenpaar. Der indifferente Elektrodenpol ist von einer Ringelektrode gebildet, die die Stimulationselektrode wenigstens teilweise umschließt. Alternativ kann der indifferente Elektrodenpol auch von einer anderen gegenüber der Stimulationselektrode großflächigeren Elektrode in der Nähe der Stimulationselektrode gebildet sein.

Die Stimulationselektrode ist eine Helixelektrode. Eine Helixelektrode erlaubt eine Befestigung des epikardialen Herzstimulators indem die Helixelektrode ähnlich eines Korkenziehers in das Myokard eingeschraubt wird.

Vorzugsweise ist der epikardiale Herzstimulator als ventrikulärer Herzschrittmacher so ausgebildet, dass er wenigstens in einem VDD-Betriebsmodus atriumsynchron zu betreiben ist.

Außerdem ist der epikardiale Herzstimulator vorzugsweise ein Demand-Schrittmacher, der ausgebildet ist, intrinsische Ereignisse einer jeweiligen zu stimulierenden Kammer zu erfassen und eine Abgabe eines Stimulationsimpulses zu unterdrücken, falls innerhalb eines entsprechenden Zeitfensters eine intrinsische Aktivität der jeweiligen Herzkammer erfasst wird.

Gemäß einer weiteren bevorzugten Ausführungsvariante ist der epikardiale Herzstimulator ein ratenadaptiver Schrittmacher, der eine Stimulationsrate an einen hämodynamischen Bedarf eines jeweiligen Patienten anpassen kann.

Die Erfindung soll nun anhand von Ausführungsbeispielen mit Bezug auf die Figuren erläutert werden. Von den Figuren zeigen:
- Fig. 1:: Eine Ansicht eines epikardialen Herzschrittmachers auf dessen nach Implantation dem Herzen zugewandter Seite;
- Fig. 2:: eine Skizze zur Erläuterung der Verschaltung der Elektroden des epikardialen Herzschrittmachers aus Fig. 1 mit einer Schrittmacher logik und Signalverarbeitung;
- Fig. 3:: ein schematisches Blockdiagramm der wesentlichen Komponenten der Schrittmacherlogik und Signalverarbeitung;
- Fig. 4a bis 4d:: verschiedene mögliche Orientierungen des epikardialen Herzschrittmachers aus Fig. 1 nach Implantation;
- Fig. 5a und 5b:: zwei alternative Ausführungsvarianten eines epikardialen Herzschrittmachers; und
- Fig. 6:: von einem epikardialen Herzschrittmacher aufgenommene extrakardiale Signale.

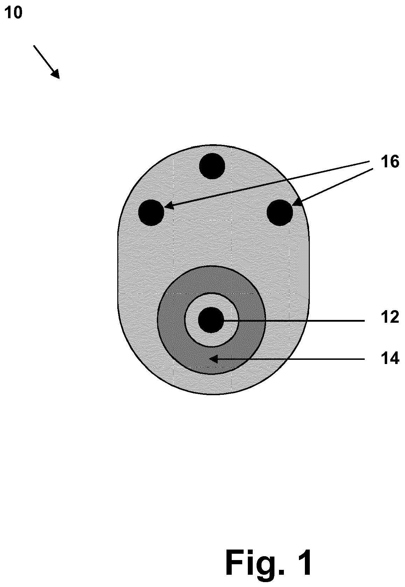

Fig. 1 zeigt eine Ansicht eines epikardialen Herzschrittmachers 10 auf dessen nach Implantation dem Herzen zugewandter Seite. Auf dieser Seite sind zum einen eine Helixelektrode 12 als differenter Stimulationselektrodenpol angeordnet und um diesen herum eine Ringelektrode 14 als indifferenter Elektrodenpol. Die Helixelektrode 12 und die Ringelektrode 14 dienen der Stimulation derjenigen Herzkammer oder Vorkammer, der benachbart des epikardialen Herzschrittmachers 10 nach Implantation angeordnet ist. In möglichst großem Abstand zur Helixelektrode 12 sind drei Sensingelektroden 16 angeordnet.

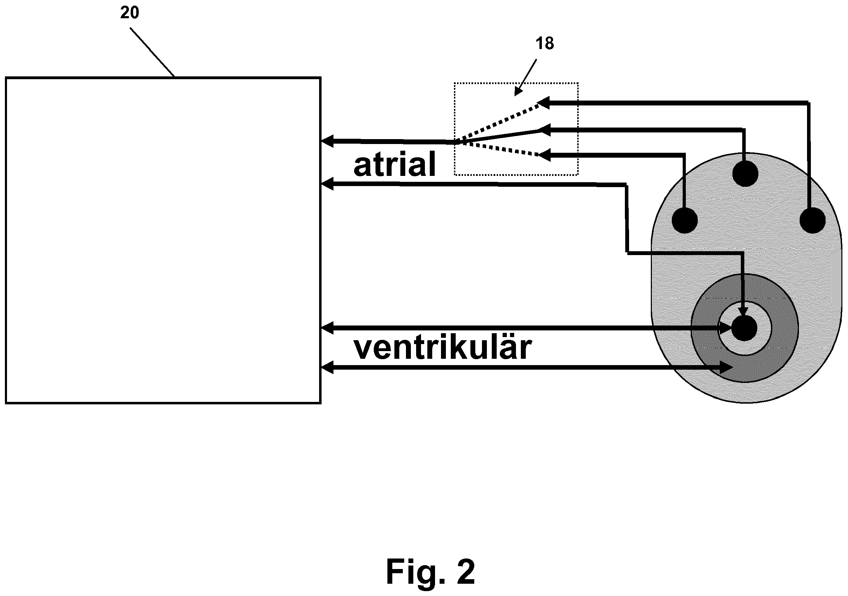

Wie Fig. 2 zu entnehmen ist, sind die drei Sensingelektroden 16 über einen programmierbaren Schalter 18 mit einer Schrittmacherlogik und Signalverarbeitung 20 verbunden. Ebenfalls mit der Schrittmacherlogik und Signalverarbeitung 20 verbunden sind die Helixelektrode 12 und die Ringelektrode 14.

Bei Verwendung des epikardialen Herzstimulators 10 als ventrikulären Herzschrittmacher bildet die Helixelektrode 12 die ventrikuläre Stimulationselektrode und die Ringelektrode 14 die entsprechende Gegenelektrode.

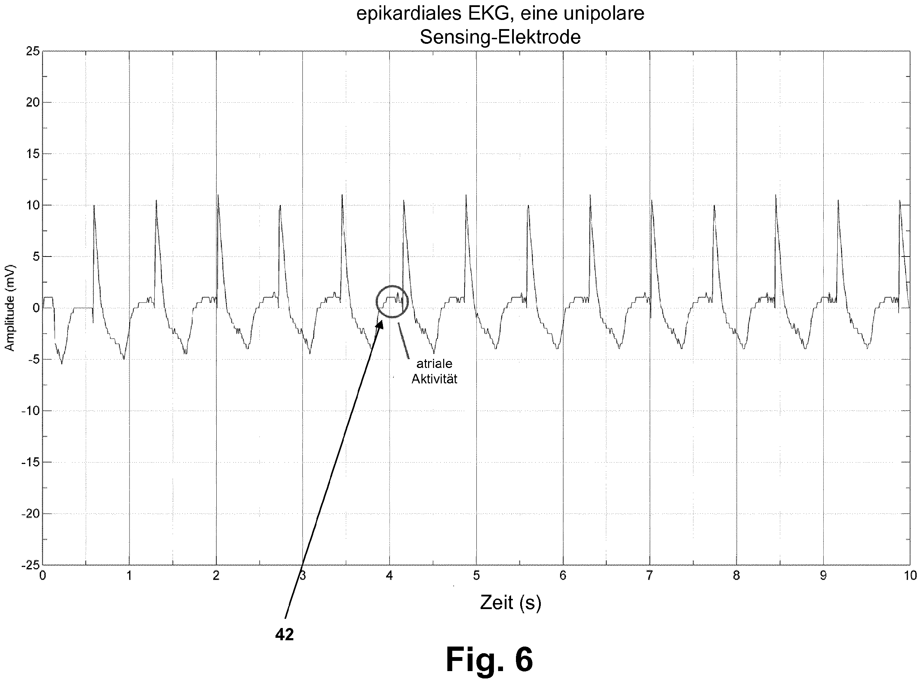

Außerdem dient die Helixelektrode 12 zusammen mit einer der Sensingelektroden 16 zum Erfassen elektrischer Potentialverläufe, die ein Fernfeld-Elektrokardiogramm repräsentieren, so wie es beispielsweise in Fig. 6 abgebildet ist. Der programmierbare Schalter 18 erlaubt es, jeweils diejenige Sensingelektrode 16 mit einer entsprechenden Fernfeld-Sensingeinheit der Schrittmacherlogik und Signalverarbeitung 20 zu verbinden, die zusammen mit der Helixelektrode 12 das beste Signal liefert.

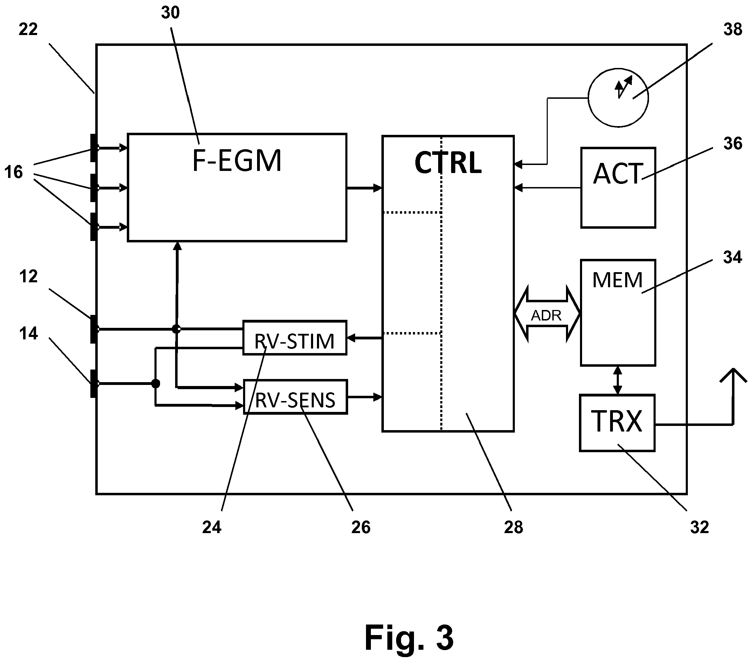

In Fig. 3 sind einige Komponenten der Schrittmacherlogik und Signalverarbeitung 20 schematisch dargestellt. Die Schrittmacherlogik und Signalverarbeitung 20 befindet sich in einem Gehäuse 22, an dessen Oberfläche die Stimulationselektrode 12, die Ringelektrode 14 sowie die drei Sensingelektroden 16 angeordnet sind.

Die Stimulationselektrode 12 und die Ringelektrode 14 sind jeweils sowohl mit einer Stimulationseinheit 24 als auch mit einer Sensingeinheit 26 verbunden. Bei Verwendung des epikardialen Herzstimulators als rechtsventrikulären Herzschrittmacher ist die Stimulationseinheit 24 eine rechtsventrikuläre Stimulationseinheit und die Sensingeinheit 26 eine rechtsventrikuläre Sensingeinheit. Die Stimulationseinheit 24 ist ausgebildet, die Energie für einen Stimulationsimpuls bereitzustellen und auf ein Auslösesignal hin über die Stimulationselektrode 12 an anliegendes Gewebe abzugeben. Hierzu ist die Stimulationseinheit 24 mit einer Stimulationssteuereinheit 28 verbunden, die ein entsprechendes Auslösesignal generiert. Die Sensingeinheit 26 kann über die Stimulationselektrode 12 und die Ringelektrode 14 lokale Herzereignisse erfassen, das heißt insbesondere solche Herzereignisse, die der Kammer zuzuordnen sind, der benachbart der epikardiale Herzstimulator 10 angeordnet ist. Im Falle eines ventrikulären Herzschrittmachers erfasst die Sensingeinheit 26 somit rechtsventrikuläre Ereignisse und gibt beim Erfassen eines jeweiligen ventrikulären Ereignisses ein entsprechendes Ausgangssignal an die Stimulationssteuereinheit 28 aus. Dies erlaubt es der Stimulationssteuereinheit 28, Stimulationsimpulse nur im Bedarfsfall auszulösen, nämlich dann, wenn die Sensingeinheit 26 kein intrinsisches ventrikuläres Ereignis erfasst hat.

Die drei Sensingelektroden 16 sind mit einer Fernfeld-Sensingeinheit 30 verbunden, die über einen weiteren Eingang mit der Stimulationselektrode 12 verbunden ist. Die Fernfeld-Sensingeinheit 30 ist ausgebildet, die Potentialdifferenz zwischen einer der drei Sensingelektroden 16 und der Stimulationselektrode im Sinne eines Fernfeld-Elektrokardiogramms auszuwerten. Dazu weist die Fernfeld-Sensingeinheit 30 einen in Fig. 3 nicht dargestellten, jedoch in Fig. 2 angedeuteten programmierbaren Schalter 18 auf, der es erlaubt, jeweils eine der drei Sensingelektroden 16 für das Sensing auszugeben und/oder zu verwenden. Es sei angemerkt, dass statt drei Sensingelektroden 16 auch nur eine einzige Sensingelektrode oder auch zwei oder mehr als drei Sensingelektroden vorgesehen sein können. Falls nur eine einzige Sensingelektrode 16 vorgesehen ist, kann der programmierbare Schalter 18 entfallen. Die Fernfeld-Sensingeinheit 30 liefert an die Stimulationssteuereinheit 28 ein Ausgangssignal, wie es beispielhaft in Fig. 6 abgebildet ist, und welches Signalmerkmale enthält, die beispielsweise eine atriale Aktivität signalisieren. Dies ist in Fig. 6 entsprechend hervorgehoben. Mit Hilfe der Fernfeld-Sensingeinheit 30 ist es der Stimulationssteuereinheit 28 somit möglich, auch beispielsweise atriale Ereignisse zu erfassen, wenn der epikardiale Herzstimulator 10 als ventrikulärer Herzschrittmacher verwendet und entsprechend, beispielsweise dem rechten Ventrikel benachbart, angeordnet ist.

Mit dem in Fig. 3 abgebildeten epikardialen Herzstimulator ist somit beispielsweise eine atriumsynchrone bedarfsabhängige Stimulation des rechten Ventrikels möglich.

In Fig. 3 ist außerdem angedeutet, dass die Stimulationssteuereinheit mit einer Speichereinheit 30 verbunden sein kann, die beispielsweise dem Speichern von Steuersignalen dient oder aber auch dem Speichern von Signalverläufen, die der epikardiale Herzstimulator 12 aufgenommen hat. Um den epikardialen Herzstimulator 10 umprogrammieren zu können oder aufgenommene Signalverläufe zur Auswertung an ein externes Gerät übertragen zu können, weist der epikardiale Herzschrittmacher 12 außerdem eine Telemetrieeinheit 34 auf, die mit dem Speicher 32 verbunden ist. Um eine dem hämodynamischen Bedarf eines jeweiligen Patienten angemessene Stimulationsrate einstellen zu können, kann die Stimulationssteuereinheit außerdem mit einem Aktivitätssensor 36 der an sich bekannten Art verbunden sein, so dass sich ein ratenadaptiver Herzschrittmacher ergibt. Der Aktivitätssensor 36 kann ein an sich bekannter Sensor sein, der ein Ausgangssignal liefert, welches vom hämodynamischen Bedarf eines Patienten abhängt. Typischerweise ist die Stimulationssteuereinheit 28 auch mit einer Zeitgebereinheit 38 verbunden.

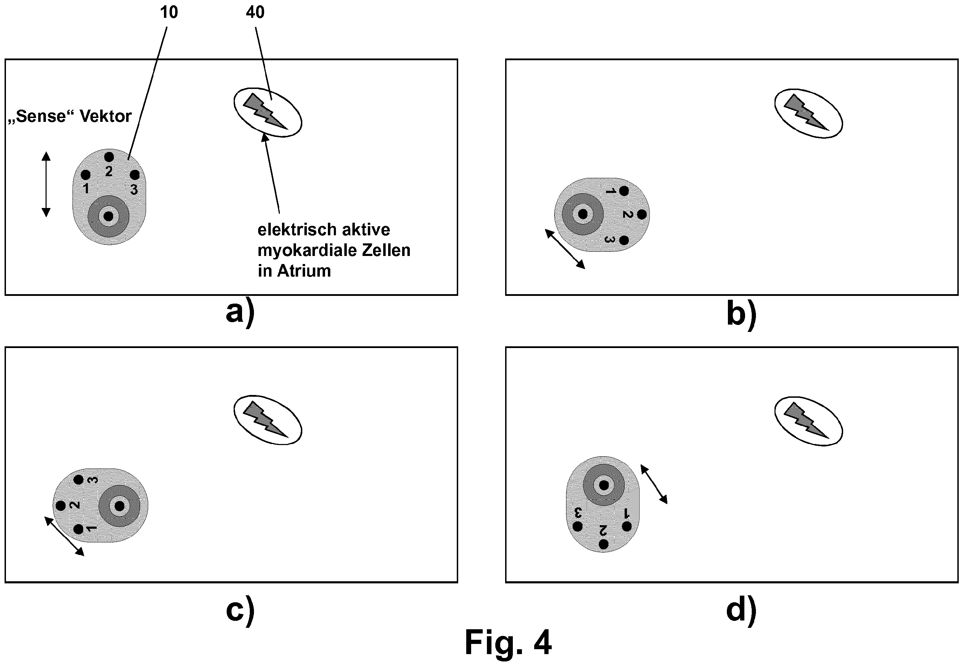

Figuren 4c) bis 4d) erläutern beispielhaft, dass die drei Stimulationselektroden 16 des epikardialen Stimulators 10 erlauben, mit einer der Sensingelektroden 16 und der Stimulationselektrode 12 jeweils einen Sensingvektor aufzuspannen, der bei verschiedenen Orientierungen des Herzstimulators 10 in Bezug auf elektrisch aktive Herzmuskelzellen 40 ein Erfassen eines aussagekräftigen Fernfeld-Elektrokardiogrammsignals erlaubt.

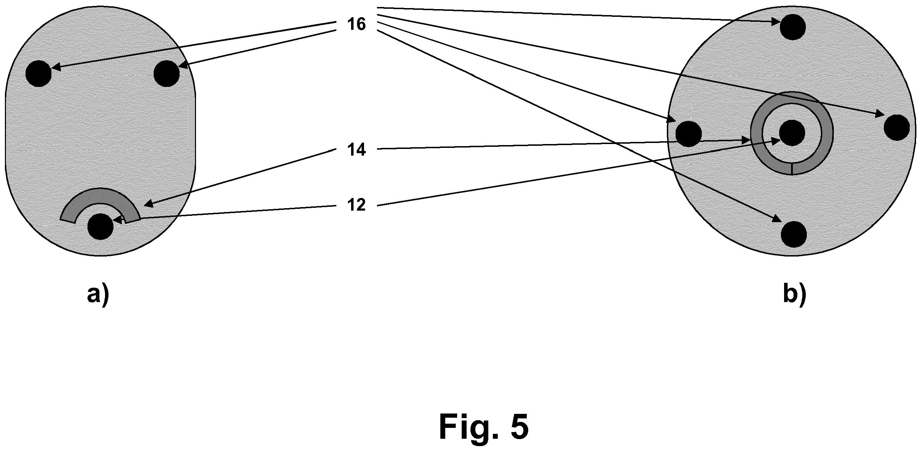

Fig. 5 erläutert, dass neben der beispielhaft in den Figuren 1 bis 4 dargestellten Anordnung von Elektroden auch alternative Elektrodenzahlen und -anordnungen vorgesehen sein können. So zeigt Fig. 5a, dass der indifferente Elektrodenpol nicht notwendigerweise von einer Ringelektrode gebildet sein muss, sondern beispielsweise auch nur von einer halben Ring- oder einer andersgearteten, etwas größeren Fläche gebildet sein kann. Außerdem können anstelle von drei Sensingelektroden 16 auch nur zwei Sensingelektroden vorgesehen sein; siehe Fig. 5a.

In Fig. 5b ist schließlich dargestellt, dass auch eine zentrale Anordnung des differenten Elektrodenpols (Helixelektrode 12) vorgesehen sein kann, mit darum herum angeordneter indifferenter Ringelektrode 14. Wiederum in möglichst großem Abstand von dem differenten Stimulationselektrodenpol 12 sind vier Sensingelektroden 16 angeordnet. Mit einer Konfiguration wie der in Fig. 5b lässt sich noch eine größere Zahl unterschiedlicher Sensingvektoren bilden. Insbesondere kann ein Sensing auch über zwei der vier Sensingelektroden erfolgen. In diesem Falle ist die Fernfeld-Sensingeinheit nicht mit einer Sensingelektrode und dem differenten Stimulationselektrodenpol verbunden, sondern mit zwei Sensingelektroden.

In Figur 6 ist beispielhaft ein extrakardiales Elektrokardiogramm dargestellt, wie es mit einem epikardialen Herzstimulator 10 aufgenommen werden kann. Beispielhaft ist ein Signalabschnitt 42 markiert, der ein atriales Ereignis repräsentiert.

### Bezugszeichenliste

- 10: Herzschrittmacher
- 12: Stift- oder Helixelektrode
- 14: Ringelektrode
- 16: Sensingelektrode
- 18: Schalter
- 20: Signalverarbeitung
- 22: Gehäuse
- 24: Stimulationseinheit
- 26: Sensingeinheit
- 28: Stimulationssteuereinheit
- 30: Fernfeld-Sensingeinheit
- 32: Speicher
- 34: Telemetrieeinheit
- 36: Aktivitätssensor
- 38: Zeitgebereinheit
- 40: elektrisch aktive myokardiale Zellen
- 42: atriales Ereignis im fernfeld-Elektrokardiogramm

## Patentansprüche

1. Epikardialer Herzstimulator (10) mit einem Gehäuse (20) und in diesem Gehäuse (20) angeordneten elektrischen Komponenten, die eine Stimulationseinheit (24) und eine mit der Stimulationseinheit (24) verbundenen Stimulationssteuereinheit (28) umfassen, sowie mit wenigstens einer Stimulationselektrode (12) an dem Gehäuse (20), die mit der Stimulationseinheit (24) verbunden ist, die Stimulationseinheit (24) ausgebildet ist, elektrische Energie für einen Stimulationsimpuls zur Verfügung zu stellen und auf ein entsprechendes Auslösesignal der Stimulationssteuereinheit (28) hin einen Stimulationsimpuls über die Stimulationselektrode abzugeben, und wobei an dem Gehäuse (20) eine Sensingelektrode (16) zum Abfühlen elektrischer Potentiale vorgesehen ist, die mit einer Sensingeinheit (30) in dem Gehäuse (20) elektrisch verbunden ist, wobei die Sensingeinheit (30) ihrerseits wiederum mit der Stimulationssteuereinheit (28) verbunden ist und die Stimulationssteuereinheit (28) so konfiguriert ist, dass sie in einem entsprechenden Betriebsmodus das Abgeben eines jeweiligen Auslösesignals in Abhängigkeit eines Ausgangssignals der Sensingeinheit (30) steuert,
**dadurch gekennzeichnet, dass**
die Stimulationselektrode (12), der indifferente Elektrodenpol (14) und die Sensingelektroden (16) auf der nach der Implantation des Epikardialen Herzstimulators dem Herzen zugewandten Seite angeordnet sind, wobei die Stimulationselektrode (12) als Helixelektrode zur Befestigung des epikardialen Herzstimulators ausgeführt ist; der indifferente Elektrodenpol (14) von einer Ringelektrode gebildet ist, der die Stimulationselektrode umschließt; und 4 Sensingelektroden (16) zum Erfassen elektrischer Potentialverläufe, die ein Fernfeld-Elektrokardiogramm repräsentieren, um die Stimulationselektrode (12), außerhalb des ringförmigen indifferenten Elektrodenpols (14) angeordnet sind, wobei die Erfassung elektrischer Potentialverläufe, die ein Fernfeld-Elektrokardiogramm repräsentieren, wahlweise entweder über 2 der 4 Sensingelektroden (16) oder über die Stimulationselektrode (12) und eine der Sensingelektroden (16) erfolgt.

2. Epikardialer Herzstimulator nach Anspruch 1, **dadurch gekennzeichnet, dass** die Sensingeinheit (30) mit der Sensingelektrode (16) und der Stimulationselektrode (12) oder einer zweiten Sensingelektrode (16) verbunden ist und die Stimulationssteuereinheit (28) ausgebildet ist, das Ausgangssignal der Sensingeinheit (30) hinsichtlich solcher Signalmerkmale auszuwerten, die eine Kontraktion einer entfernten Vorkammer und/oder Herzkammer anzeigen.

3. Epikardialer Herzstimulator nach Anspruch 1 oder 2, **dadurch gekennzeichnet, dass** an dem Gehäuse (20) mehrere Sensingelektroden (16) zum Abfühlen elektrischer Potentiale vorgesehen sind und dass die Sensingeinheit (30) einen Schalter umfasst, mit dem eine oder zwei der Sensingelektroden (16) mit den übrigen zum Aufnehmen eines Elektrokardiogramm ausgebildeten Komponenten der Sensingeinheit (30) umschaltbar elektrisch zu verbinden sind.

4. Epikardialer Herzstimulator nach wenigstens einem der Ansprüche 1 bis 3, **dadurch gekennzeichnet, dass** an dem Gehäuse (20) ein indifferenter Elektrodenpol (14) in unmittelbarer Nähe der Stimulationselektrode (12) angeordnet ist.

5. Epikardialer Herzstimulator nach wenigstens einem der Ansprüche 1 bis 4, **dadurch gekennzeichnet, dass** der epikardiale Herzstimulator (10) als ventrikulärer Herzschrittmacher so ausgebildet ist, dass er wenigstens in einem VDD-Betriebsmodus atriumsynchron zu betreiben ist.

6. Epikardialer Herzstimulator nach wenigstens einem der Ansprüche 1 bis 5, **dadurch gekennzeichnet, dass** der epikardiale Herzstimulator (10) ein Demand-Schrittmacher ist.

7. Epikardialer Herzstimulator nach wenigstens einem der Ansprüche 1 bis 6, **dadurch gekennzeichnet, dass** der epikardiale Herzstimulator (10) ein ratenadaptiver Schrittmacher ist.

## Claims

1. An epicardial heart stimulator (10), comprising a housing (20) and electrical components arranged in this housing (20), which comprise a stimulation unit (24) and a stimulation control unit (28) connected to the stimulation unit (24), and further comprising, on the housing (20), at least one stimulation electrode (12), which is connected to the stimulation unit (24), the stimulation unit (24) being designed to provide electrical energy for a stimulation pulse and to emit a stimulation pulse via the stimulation electrode in response to a corresponding trigger signal of the stimulation control unit (28), and a sensing electrode (16), which is electrically connected to a sensing unit (30) in the housing (20), being provided on the housing (20) for sensing electrical potentials, the sensing unit (30) in turn being connected to the stimulation control unit (28), and the stimulation control unit (28) being configured, in an appropriate operating mode, to control the emission of a respective trigger signal as a function of an output signal of the sensing unit (30),
**characterized in that**
the stimulation electrode (12), the indifferent electrode pole (14) and the sensing electrodes (16) are arranged on the side facing the heart after implantation of the epicardial heart stimulator, the stimulation electrode (12) being designed as a helix electrode for attachment of the epicardial heart stimulator, the indifferent electrode pole (14) being formed by a ring electrode surrounding the stimulation electrode, and 4 sensing electrodes (16) for detecting electrical potential profiles representing a far-field electrocardiogram being arranged around the stimulation electrode (12), outside the annular indifferent electrode pole (14), the detection of electrical potential profiles representing a far-field electrocardiogram selectively taking place either by way of 2 of the 4 sensing electrodes (16) or by way of the stimulation electrode (12) and one of the sensing electrodes (16).

2. The epicardial heart stimulator according to claim 1, **characterized in that** the sensing unit (30) is connected to the sensing electrode (16) and the stimulation electrode (12) or a second sensing electrode (16), and the stimulation control unit (28) is designed to evaluate the output signal of the sensing unit (30) with respect to signal features that indicate a contraction of an atrium and/or a ventricle lying furthest away.

3. The epicardial heart stimulator according to claim 1 or 2, **characterized in that** a plurality of sensing electrodes (16) for sensing electrical potentials are provided on the housing (20), and the sensing unit (30) comprises a switch by which one or two of the sensing electrodes (16) are to be electrically connected, so as to be switchable, to the remaining components of the sensing unit (30) that are designed to record an electrocardiogram.

4. The epicardial heart stimulator according to at least one of claims 1 to 3, **characterized in that** an indifferent electrode pole (14) is arranged on the housing (20) in the immediate vicinity of the stimulation electrode (12).

5. The epicardial heart stimulator according to at least one of claims 1 to 4, **characterized in that** the epicardial heart stimulator (10) is designed as a ventricular cardiac pacemaker so as to be operated atrial-synchronously at least in a VDD operating mode.

6. The epicardial heart stimulator according to at least one of claims 1 to 5, **characterized in that** the epicardial heart stimulator (10) is a demand pacemaker.

7. The epicardial heart stimulator according to at least one of claims 1 to 6, **characterized in that** the epicardial heart stimulator (10) is a rate-adaptive pacemaker.

## Revendications

1. Stimulateur cardiaque épicardique (10) doté d'un boîtier (20) et des composants électriques disposés dans ce boîtier (20), qui comprennent une unité de stimulation (24) et une unité de commande de stimulation (28) reliée à l'unité de stimulation (24), doté également d'au moins une électrode de stimulation (12) au niveau du boîtier (20) qui est reliée avec l'unité de stimulation (24), l'unité de stimulation (24) est conçue pour mettre à disposition de l'énergie électrique pour une impulsion de stimulation et émettre une impulsion de stimulation suite à un signal de déclenchement correspondant de l'unité de commande de stimulation (28) par le biais de l'électrode de stimulation, et où une électrode de détection (16), qui est reliée électriquement avec une unité de détection (30) dans le boîtier (20), est prévue au niveau du boîtier (20) pour la détection de potentiels électriques, où l'unité de détection (30), pour sa part, est de nouveau reliée avec l'unité de commande de stimulation (28) et l'unité de commande de stimulation (28) est configurée de manière à ce qu'elle commande, dans un mode de fonctionnement approprié, l'émission d'un signal de déclenchement respectif en fonction d'un signal de sortie de l'unité de détection (30),
**caractérisé en ce que**
l'électrode de stimulation (12) le pôle d'électrode indifférent (14) et les électrodes de détection (16) sont disposés sur le côté du cœur orienté vers l'implantation du stimulateur cardiaque épicardique, où l'électrode de stimulation (12) est conçue sous forme d'électrode en hélice pour la fixation du stimulateur cardiaque épicardique ; le pôle d'électrode indifférent (14) est formé par une électrode annulaire qui entoure l'électrode de stimulation ; et 4 électrodes de détection (16), pour la détection d'évolutions de potentiels électriques qui représentent un électrocardiogramme à champ lointain, sont disposées autour de l'électrode de stimulation (12), à l'extérieur du pôle d'électrode indifférent (14) en forme d'anneau, où la détection des évolutions de potentiels électriques qui représentent un électrocardiogramme à champ lointain a lieu, au choix, soit par le biais de 2 des 4 électrodes de détection (16), soit par le biais de l'électrode de stimulation (12) et d'une des électrodes de détection (16).

2. Stimulateur cardiaque épicardique selon la revendication 1, **caractérisé en ce que** l'unité de détection (30) est reliée avec l'électrode de détection (16) et l'électrode de stimulation (12), ou avec une deuxième électrode de détection (16), et l'unité de commande de stimulation (28) est conçue pour évaluer le signal de sortie de l'unité de détection (30) en ce qui concerne celles des caractéristiques de signal qui indiquent une contraction d'une préchambre et/ou d'un ventricule éloignés.

3. Stimulateur cardiaque épicardique selon la revendication 1 ou la revendication 2, **caractérisé en ce que** plusieurs électrodes de détection (16) sont prévues au niveau du boîtier (20) pour la détection de potentiels électriques et que l'unité de détection (30) comprend un commutateur avec lequel une ou deux des électrodes de détection (16) sont à relier électriquement de manière réversible avec les autres pour l'enregistrement de composants de l'unité de détection (30) conçus pour l'enregistrement d'un électrocardiogramme.

4. Stimulateur cardiaque épicardique selon au moins une des revendications 1 à 3, **caractérisé en ce qu'**un pôle d'électrode indifférent (14) est disposé à proximité immédiate de l'électrode de stimulation (12) au niveau du boîtier (20).

5. Stimulateur cardiaque épicardique selon au moins une des revendications 1 à 4, **caractérisé en ce que** le stimulateur cardiaque épicardique (10) est conçu sous forme de stimulateur cardiaque ventriculaire de manière à ce qu'il puisse fonctionner de manière synchrone avec l'atrium dans un mode de fonctionnement VDD.

6. Stimulateur cardiaque épicardique selon au moins une des revendications 1 à 5, **caractérisé en ce que** le stimulateur cardiaque épicardique (10) est un stimulateur basé sur la demande.

7. Stimulateur cardiaque épicardique selon au moins une des revendications 1 à 6, **caractérisé en ce que** le stimulateur cardiaque épicardique (10) est un stimulateur à fréquence asservie.
